# EUROPEAN PATENT APPLICATION

(11) **EP 2 191 850 A1**
(43) Date of publication of application: **02.06.2010**
(21) Application number: 08831885.2
(22) Date of filing: 19.09.2008
(51) Int. Cl.: A61L 27/00, A61L 29/00

(54) **TISSUE-BONDABLE MATERIAL FOR MEDICAL USE**

(30) Priority: 21.09.2007 JP 2007245401
(71) Applicant: Kyushu University, National University Corporation, Higashi-ku Fukuoka-shi Fukuoka 812-8581 (JP)
(72) Inventor: ISHIKAWA, Kunio, Fukuoka-shi Fukuoka 812-8581 (JP)
(74) Representative: HOFFMANN EITLE
(86) International application number: PCT/JP2008/066994
(87) International publication number: WO 2009/038180

(57) **Abstract**

The present invention relates to a medical tissue-binding material, especially a soft tissue-binding material capable of attaching to a soft biological tissue such as a bone reconstruction material or a transdermal terminal, and a method for preparation thereof. In particular, the present invention relates to a medical tissue-binding material which comprises a base material having calcium binding onto the surface, provided that the base material is not titanium or titanium alloy. Also, the present invention relates to a method for preparing a medical tissue-binding material which comprises soaking a base material into a calcium ion containing solution. Introduction of at least one group selected from the group consisting of hydroxyl, carboxyl, sulfonate, amino, silanol and phosphate to the surface of the base material is effective for said method.

## Description

### Technical Field

The present invention relates to a medical tissue-binding material and a method for preparation thereof. Particularly, it relates to a medical tissue-binding material, in particular a bone reconstruction material applicable to the reconstruction of bone tissue; or a soft tissue-binding material capable of attaching to a soft biological tissue such as a transdermal terminal, and a method for preparation thereof.

### Background Art

In the medical field, there are many cases requiring reconstruction of bone defects caused by pathogeny or injury. There are also many cases requiring the communication of the inside of a body to the outside through an artificial material, for example, due to the necessity of peritoneal dialysis or energy transmission to an artificial organ. In those cases, a medical tissue-binding material capable of binding to a bone tissue or a soft biological tissue is needed.

As a bone reconstruction material, there are exemplified a stem, a bone plate, a fixation screw, etc. used for the art of replacement with alternative bone. Other examples include a fixture used for dental implant. These bone reconstruction materials, which are desired to bind to a bone tissue as soon as possible, are made of titanium, Co-Cr alloy, Ni-Cr alloy, stainless steel, alumina, zirconia, apatite, AW-glass ceramics, carbon, polylactate, polyethylene terephthalate, polyethylene, polypropylene, etc. depending on the intended use.

Up to this time, titanium has been commonly used as a bone reconstruction material, because it binds to bone within a relatively short period of time. But, the binding rate of titanium to a bone tissue is still not sufficient. Therefore, titanium is coated by apatite on the surface for promoting its binding rate to bone (cf. Patent Reference 1). Apatite coating is effective in promotion of the binding rate to bone, but its detachment with time is inevitable so that a desired clinical result over a long period of time can often hardly be obtained.

As an example of the artificial material for communicating the inside of a body to the outside, there is a transdermal terminal. For this purpose, silicone is mainly used, because of its good tissue affinity. However, silicone is not capable of binding to a soft biological tissue so that the epidermal cells invaginate from the interface of the tissue with silicone to give voids between the tissue and silicone (downgrowth), through which a bacterial infection is caused (cf. Non-patent Reference 1).

Accordingly, a medical tissue-binding material is desired to have both of 1) a good affinity to a tissue and 2) a good binding property to a tissue, the tissue including a bone tissue and/or a soft biological tissue. The tissue affinity is essential to a medical tissue-binding material and can be confirmed by observing macroscopically or histopathologically an inflammatory lesion in laboratory animals implanted the medical tissue-binding material. It may be also confirmed by the initial adhesion or growth of cells. The tissue binding ability is a binding property of a medical tissue-binding material to a tissue when such material is implanted. The tissue binding ability is a highly important factor for a bone reconstruction material, a transdermal terminal, etc. and affects significantly on the clinical results. It can be determined by the histopathological observation of laboratory animals implanted a medical tissue-binding material. It may be also determined by the initial adhesion or growth of cells.

It is well known that the binding ability of a medical tissue-binding material to a bone tissue can be assessed by using a simulated body fluid (cf. Patent References 1-4). It is also known that a material capable of promoting the differentiation of osteoblast shows a good bone binding property.

As to the binding property of a medical material to a tissue, there are used various terms. For example, the term "bioactive material" is used for a material capable of binding to a bone tissue. The term "osteoconductive material" is used for a medical material capable of binding to a bone tissue without intervention of a fibrous binding tissue at a level of the electron microscopic observation. The term "bone binding material" is used for a medical material capable of binding functionally to a bone tissue. The term "tissue-binding material" herein used covers all the terms as above exemplified, inclusively.
Patent Reference 1: JP-H10-179718;
Patent Reference 2: JP-2000-93498;
Patent Reference 3: JP-2000-116673;
Patent Reference 4: JP-2002-102330;
Non-patent Reference 1: "Surface and Interface Technology" Volume 2, Application (Kenichi Honda), Chapter 11: Surface and interface technology for biocompatible materials; <2-2> Soft tissue, page 205 (2005).

### Disclosure of Invention

### Problem to be solved

Titanium is an only material for bone reconstruction which has been found to show a bone binding ability in a narrow sense, i.e. an only medical material capable of binding functionally to a bone tissue, although a fibrous binding tissue is observed between such material and a bone tissue by an electron microscope. For enhancing the bone forming rate of titanium, it is believed to be effective to treat it with a calcium ion containing solution. It is also believed that this method is applicable only to titanium, which has been considered as an only bone-binding material, because the surface potential of titanium is negative and the binding property of titanium to calcium is essential. From these points of view, it has not been studied to apply the above method to various base materials other than titanium, despite of the intensive studies for the clinical application of them.

Further, even when titanium or titanium alloy is used as a base material, there have been the following problems: the amount of calcium capable of binding to the surface of titanium or titanium alloy is limited so that enhancement of the bone forming rate is restricted; detachment of calcium binding to the surface of such titanium material with time is inevitable so that the long-term clinical effect is hardly expected, etc. In order to overcome these problems, there has been a strong demand to development of a method for coating said titanium material with a larger amount of calcium more firmly.

Silicone is presently the most commonly used for a transdermal terminal. However, silicone does not have a binding property to a soft biological tissue, and epidermal cells cause downgrowth into the body to make voids between silicone and the tissue, leading bacterial infection. To solve this problem, a transdermal terminal comprising tissue-binding apatite having a good tissue-binding property has been developed. However, there is still a need for a more effective transdermal terminal with apatite enhanced in tissue-binding property, which shows good flexibility and tissue-binding property.

Accordingly, the object of the present invention is to provide a medical tissue-binding material which is satisfactory in showing significant tissue affinity and tissue-binding property.

### Means to solve problem

As a results of an extensive study, it has now been found that a medical tissue-binding material having the desired properties as above can be obtained by successful binding of calcium to the surface of a base material even when it is other than titanium or titanium alloy.

In one aspect of the present invention, there are provided a medical tissue-binding material having calcium binding to the surface of a base material and a method for preparation thereof. The term "binding" as used in the context between calcium and base material, is intended to mean a state or condition that elemental calcium or a compound including calcium is held firmly on the surface of a base material. Binding through chemical bonding is one of preferred examples, and the chemical bonding may be achieved by reacting hydroxyl groups at the surface of an alumina plate with calcium ion to make calcium chemically bound to said surface.

Metal materials such as Co-Cr alloy, Ni-Cr alloy and stainless steel have been clinically applied to biomaterials for loading parts due to their good mechanical properties and are preferably used as the base material in the present invention.

Ceramic materials such as alumina and zirconia have been clinically applied to alternate materials for slide parts due to their good tissue affinity and abrasion resistance and are preferably used as the base material in the present invention. Since alumina and zirconia has no bone-binding ability, they have been fitted to bone tissues via the mechanical fitting force by forming the surface asperity. Therefore, it is desirable to provide bone-binding alumina or zirconia.

Among ceramic materials, apatite, AW-glass ceramics, etc. have a bone-binding ability and are used as bone prosthesis. Despite their bone-binding ability, it is still desired to increase their bone-binding ability. Those are understood to be preferably used as the base material in the present invention.

Carbon as one of ceramic materials is used, for example, for spinal reconstruction in a cage form due to its radiolucency. Autogenous bone is charged in the cage to allow binding to a bone tissue, but it is still desired to provide carbon with bone-binding ability. Carbon is also preferred as the base material in the present invention.

A polymer material such as polyethylene terephthalate silk is clinically used as an artificial tendon, and it is preferably used as the base material in the present invention. The end of tendon is required to bind to a bone, but polyethylene terephthalate does not have a bone-binding ability. Because of this reason, It has been bound to bone by the use of a screw. It is thus desirable to provide a polymer material with a bone-binding ability.

### Effect of invention

Surprisingly, it has now been found that calcium can be bound to the surface of a material other than titanium considered to be as an only material showing bone-binding ability in a narrow sense to provide such other material with remarkable tissue affinity and tissue-binding ability, particularly bone-binding ability.

### Brief Description of Drawings

Fig. 1: showing the XPS patterns of the surfaces of the medical tissue-binding materials prepared in Examples 1 and 2 and of the alumina plates of Comparative Examples 1 and 2.
Fig.2: showing the infrared spectra of the precipitates formed on the surfaces of the medical tissue-binding materials prepared in Examples 1 and 2 by soaking those materials in a simulated body fluid for 14 days.

### Best Mode for Carrying Out the Invention

The present invention relates to a medical tissue-binding material having calcium binding to the surface of a base material (provided that the base material is not titanium or titanium alloy), which is prepared by a method comprising a step of soaking the base material in a calcium ion containing solution. Binding of calcium to the surface of the base material is preferably achieved by chemical bonding. It is effective in achievement of such chemical bonding to subject previously the base material to treatment for introduction of least one functional group selected from the group consisting of hydroxyl, carboxyl, sulfonate, amino, silanol and phosphate groups onto the surface of the base material. The introduction of said functional groups may be accomplished by a per se conventional procedure such as ozone treatment for introduction of hydroxyl or carboxyl, sulfur triozide gas treatment for introduction of sulfonate, aminolysis for introduction of amino, group, silane treatment for introduction of silanol, phosphoric acid treatment for introduction of phosphate, etc. Introduction of functional groups as above aims at binding of calcium to the surface of a base material by the chemical bonding, and it is natural from the theoretical viewpoint to carry out treatment for binding of calcium subsequently, for example, by soaking of the base material in a solution containing calcium ion. However, it is also possible procedurally to carrying out introduction of the functional groups and binding of calcium simultaneously. For example, the base material may be soaked in an aqueous solution of calcium chloride in which ozone is dissolved. In this case, the operation is performed in a single step, but the reaction would proceed first with the introduction of hydroxyl groups into the surface of the base material and then with calcium binding to said surface though the hydroxyl groups. In this way, the present invention provides a medical tissue-binding material showing significant tissue affinity and tissue binding ability.

In another aspect of the present invention, there is provided a medical tissue-binding material prepared by introducing at least one kind of functional groups selected from the group consisting of hydroxyl, carboxyl, sulfonate, amino, silanol and phosphate ointo the surface of a base material selected from the group consisting of titanium and titanium alloy and then soaking the base material into a calcium ion containing solution and a method for preparing the medical tissue-binding material as above. Introduction of functional groups as above aims at binding of calcium to the surface of a base material by the chemical bonding, and it is natural from the theoretical viewpoint to carry out treatment for binding of calcium subsequently, for example, by soaking of the base material in a solution containing calcium ion. However, it is also possible procedurally to carrying out introduction of the functional groups and binding of calcium simultaneously. For example, titanium or titanium alloy as the base material may be soaked in an aqueous solution of calcium chloride in which ozone is dissolved. In this case, the operation is performed in a singe step, but the reaction would proceed first with the introduction of hydroxyl groups into the surface of titanium or titanium alloy and then with calcium binding to said surface though the hydroxyl groups.

The base material as used herein includes preferably medical materials such as Co-Cr alloy, Ni-Cr alloy and stainless steel, ceramic materials such as alumina, zirconia, apatite, AW-glass ceramics and carbon, polymer materials such as polylactate, polyethylene terephthalate, polyethylene, polypropylene and silicon, etc. No limitation is present on the shape of the base material, and any optional shape may be chosen depending on the intended use.

The calcium ion containing solution may be prepared by dissolving or suspending a calcium compound in a solvent. As the solvent, there may be used anyone which is capable of dissolving the calcium compound therein. The most preferred solvent is water, but it is possible to use as the solvent a mixture of water with a polar solvent such as an alcohol (e.g. methanol, ethanol, isopropanol) or the polar solvent itself. In the preparation of the medical tissue-binding material of the present invention, it is needed to bind a sufficient amount of calcium to the surface of the base material. However, such amount is very small, and the calcium concentration in the solution may be so low as 0.1 to 1000 mM, preferably 1 to 500 mM, more preferably 5 to 200 mM.

The calcium compound as used herein refers to any compound containing calcium, of which examples are metal calcium, calcium hydroxide, calcium carbonate, calcium chloride, calcium acetate, calcium benzoate, calcium fluoride, calcium formate, calcium gluconate, calcium hydride, calcium iodide, calcium lactate, apatite, tricalcium phosphate, tetracalcium phosphate, calcium hydrogen phosphate, calcium silicate, etc. The calcium compound may be a single calcium compound or a mixture of two or more calcium compounds.

The medical tissue-binding material of the present invention having calcium binding to the surface is prepared by soaking a base material into a calcium ion-containing solution. No limitation is present on the temperature, and the optimal temperature may be appropriately determined. For instance, soaking may be made at a temperature of 10 to 400°C. In case of the temperature being less than 50°C, the effect of treatment is sometimes restrictive, and therefore it is preferred to carry out the treatment at 50 to 350°C to give a medical tissue-binding material of higher function. Usually, soaking of the base material in a calcium ion-containing solution is carried out at 90 to 300°C, more preferably 110 to 250°C, for 1 hour to 21 days, preferably 12 hours to 14 days.

Generally, the amount of calcium binding to the surface may be increased by treating for a longer period of time, at a higher temperature, in a solution containing calcium in a higher concentrate and/or by refreshing a calcium ion-containing solution during soaking; however these result in increase of the cost. Adoption of a higher temperature may make it possible to bind calcium to the surface of the base material within a shorter period of time; however it causes the rise of the cost for manufacturing facilities.

When the medical tissue-binding material having calcium binding to the surface is prepared by soaking a base material in a calcium-containing solution at a temperature above the boiling point of said solution, adoption of the treatment under the hydrothermal condition is favorable. The treatment under the hydrothermal condition means to carry out soaking in a calcium-containing solution at a temperature higher than the boiling point of the solution. Since the solution boils at the boiling point, the treatment is typically carried out in a sealed reaction vessel such as an autoclave reactor under superheating. Because of this reason, the pressure in the reaction vessel may become more than 1 atom.

In the present invention, it is essential that calcium binds to the surface of a base material. In order to make calcium binding efficiently, it is sometimes significantly effective to introduce at least one group selected from the group consisting of hydroxyl, carboxyl, sulfonate and amino onto the surface of the base material. Even when the introduction of functional groups is not carried out, any functional groups such as hydroxyl may be originally present on the surface of a base material, and calcium may bind to such surface via those functional groups. Since the amount of the functional groups originally present is limited, the introduction of functional groups aiming at achieving an efficient binding of calcium onto the surface is effective. However, application of the introduction of functional groups could increase the cost, and therefore the skilled person may determine whether the application of such procedure is made or not depending upon the circumstances.

At least one functional groups chosen from hydroxyl, carboxyl, sulfonate and amino groups may be introduced onto the surface of a base material by a per se known conventional procedure, which includes reaction with an oxidizing agent such as ozone or potassium permanganate for introduction of hydroxyl groups, irradiation of electron rays in the presence of air for introduction of hydroxyl or carboxyl groups, plasma irradiation in the presence of ammonia or hydrogen and nitrogen for introduction of amino groups, reaction with a diamine (e.g. ethylenediamine) for introduction of amino groups, reaction with sulfuric acid for introduction of sulfate groups, etc. An optimum procedure may be chosen depending on the functional groups to be introduced.

X-ray photoemission spectroscopy (XPS) can be used to determine the bond of calcium in the obtained medical tissue-binding material characterized by calcium binding to the surface of the material (the material of the present invention). When XPS is performed for the material of the present invention using 8 kV, 30 mA of MgK-alfa-ray as X-ray, the presence of calcium can be determined around 340 to 360 eV as one or more doublet. Plotting with eV on the x-axis and CPS on the y-axis, the area of calcium from baseline is preferably 500 to 10000 eV*CPS, more preferably 1000 to 7500 eV*CPS, still more preferably 2000 to 5000 eV*CPS or more. If the base material itself contains calcium, the presence of calcium bond can be assessed by comparing XPS peaks of the other element such as phosphoric acid. The "binding" of calcium to the base material can be determined by XPS after soaking the material of the present invention into purified water for 14 days at 37 °C to dissolve calcium attached to it, wherein the calcium peak within the above range of area of the peak indicates the presence of calcium binding to the material.

The tissue-binding ability of the material of the present invention can be determined by the assessment of bone binding property by using simulated body fluid, for example according to the method described in JP-H10-179718, JP-2000-93498, JP-2000-116673 and JP-2002-102330. Briefly, after soaking the obtained material into simulated body fluid which contains inorganic ions at the analogous concentrate to body fluid, the presence and amount of precipitate of apatite can be determined as an indication the binding rate to bone. In a preferred embodiment, bone-like apatites may precipitate onto the material of the present invention within 14 days after soaking it to simulated body fluid. Such material of the present invention may show significant tissue-binding ability. Preferably, after soaking to the simulated body fluid for 14 days, more than 1 %, more preferably more than 3 %, further preferably 10 % of area of the material is covered by bone-like apatite.

Generally, the material showing remarkable tissue-binding ability also shows remarkable tissue affinity.

The material of the present invention may use for the treatment of the conditions desired to be treated by a medical material, for example treatment of osteoporosis, bone reconstruction associated with tumorectomy, replacement by alternative femoral head, replacement by alternative joint, catheter-based therapy required for dialysis or the like. The term treatment, as used herein, refers to for example surgical implantation of the material of the present invention to a defective part or part desired to be implanted. By treating those conditions with the material of the present invention, those conditions may be ameliorated, improved or arrested or the progression of those conditions may be slowed.

By treating those conditions with the material of the present invention, those conditions can be treated. In the replacement by alternative femoral head, the increased binding rate to bone may not only shorten the length of the period of the therapy, but also increase the continuousness of the therapeutic effect. In the case of a transdermal terminal, an infection can be prevented and its therapeutic effect will increase.

### Examples

The present invention will be hereinafter illustrated by way of Examples and Comparative Examples without limiting the scope of the invention thereto.

### General condition

In the method for manufacturing the medical tissue-binding materials of Examples and the materials of Comparative Examples, the treatment is performed under the hydrothermal condition, when the temperature for treatment is above 100 °C,

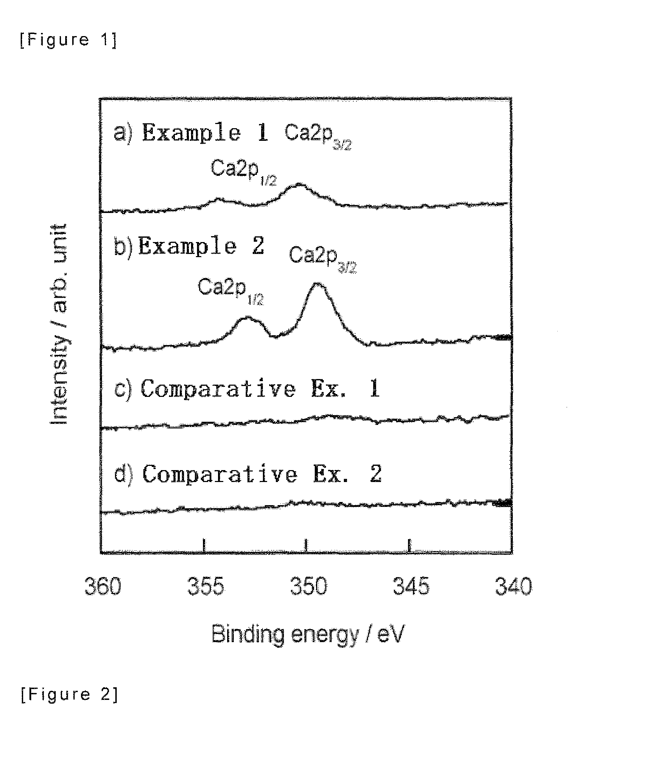

The binding of calcium to the surface of the base material is assessed by using an XPS instrument (ESCA750, Shimadzu Corporation, measurement with MgK-alfa-ray of 8 kV and 30 mA as X-ray). For the material of the present invention made of a calcium containing material, the presence of calcium binding is confirmed by the ratio of XPS peak area to phosphoric acid. An example in which the presence of calcium binding could be confirmed by XPS is shown in Figure 1. The products showing calcium peak similar to the above are indicated as O and those showing no calcium peak are indicated as X in the tables as hereinafter given.

The bone binding ability is assessed by using a simulated body fluid according to a per se known method described in JP-H10-179718, JP-2000-93498, JP-2000-116673, JP-2002-102330, etc. Briefly, the obtained material is soaked into a simulated body fluid having inorganic ion concentration similar to those of a body fluid, of which the formulation is shown in Table 1 and the pH is adjusted to 7.4 with trishydroxyaminomethane and 1N HCl at 37 °C, and then the presence and amount of precipitated apatite are determined as an index of the binding rate to bone. Determination was made by observation of a sample, which is coated with gold by sputtering method, using an electron scanning microscope (JSM-5400LV, JEOL Ltd.) at an accelerating voltage of 15 kV and a magnification of 2000-folds.
The proportion of the precipitate to the area of the observed field is recorded by percentage. In case of the precipitate being observed, its composition is analyzed by a Fourier transform infrared (FT-IR) spectrophotometer (SPECTRUM 2000, Perkin-Elmer; cumulated number: 8; resolution: 4 cm⁻¹).

**Table 1.**

| Ion | Simulated body fluid | Human plasma |
|---|---|---|
| Na⁺ | 142.0 | 142.0 |
| K⁺ | 5.0 | 5.0 |
| Mg²⁺ | 1.5 | 1.5 |
| Ca²⁺ | 2.5 | 2.5 |
| Cl⁻ | 148.8 | 103.0 |
| HCO³⁻ | 4.2 | 27.0 |
| HPO₄²⁻ | 1.0 | 1.0 |
| SO₄²⁻ | 0.5 | 0.5 |

For some materials, their abilities of initial adhesion, growth and differentiation are determined by using cells to assess their bone-binding ability in more details. Cells are obtained from tibial bone marrow of SD rats (male, 4 weeks old) and cultured in alfa-MEM supplemented with 20 % fetal bovine serum plus 1% antibiotic (10,000 units of penicillin and 10 mg/mL of streptomycin) in atmosphere containing 5% CO₂ at 37 °C, under 100 % humidity for 5 days, followed by removing floating cells to provide cells adhering to the cell culture dishes. Thus obtained cells are seeded onto the samples at 1 x 10⁴ cells/cm² and cultured for various periods of time. For the assessment of the initial cell adhering ability, cells are cultured on the samples for 7 hours; and that for cell growth, cells are cultured for 3, 5 and 7 days. Then, the number of cells adhering to the samples is counted. For cell counting, the samples are washed with phosphate buffered saline (PBS) to remove unbound cells, followed by leaving cells adhering to the samples with 0.25% trypsin/EDTA (2.5g/L of trypsin and 0.2 g/L of EDTA), and the cells are counted by a hemocytometer.

The differentiation ability of osteoblasts is assessed by measurement of the activity of alkaline phosphatase as a differentiation marker. The primary cultured tibial bone marrow cells from rat are suspended in alfa-MEM supplemented with 20 % fetal bovine serum, 1 % antibiotic (10,000 units of penicillin and 10 mg/mL of streptomycin), 10⁻⁸ mol/L or dexamethasone, 50 µg/mL of ascorbic acid and 10 mM of beta-glycerophosphoric acid and then seeded onto the samples at 1 x 10⁴ cells/cm². After culturing in atmosphere containing 5% CO₂ at 37°C under 100 % humidity for 3, 6 or 9 days, the samples are washed with PBS to remove unbound cells and then cells adhering to the samples are collected by using a cell scraper. The collected cells are milled and then subjected to determinatin of the ALP activity and the total amount of protein by the use of an ALP assay kit (Wako Pure Chemical Industries, Ltd.) and a BCA protein assay kit (Pierce Biotechnology, Inc., U.S.A) respectively to calculate the ALC activity/total protein value (IUcm²/mg).

### Preparation and assessment of medical tissue-binding material

### Example 1

An alumina plate (Nikkato) as a ceramic material was soaked into a 10 mM aqueous solution of calcium chloride and treated under a hydrothermal condition at 125°C for 7 days. After washing sufficiently, the alumina plate was analyzed by XPS to confirm calcium binding on the surface as shown in Figure 1. The peak area was 2300 cps*eV.

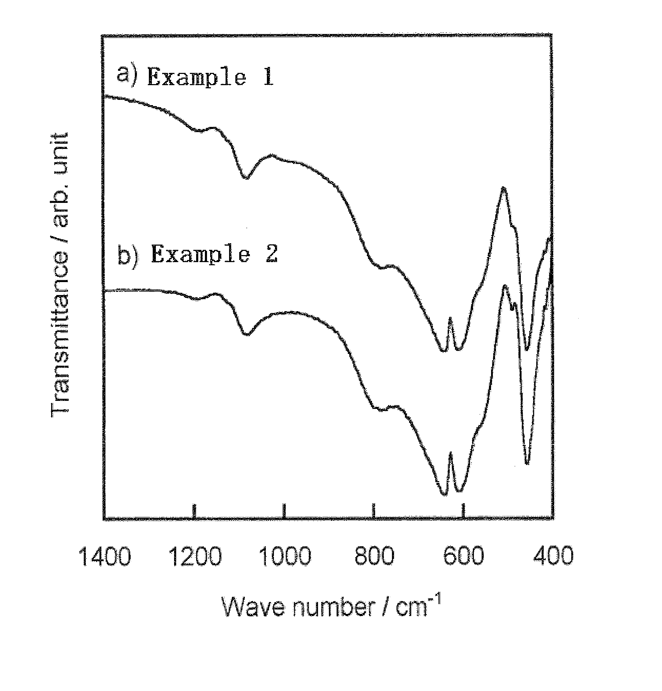

For assessing the osteoconductivity, the alumina plate having calcium binding on the surface was soaked into a simulated body fluid for 14 days. On the surface of the alumina plate, a precipitate considered to be bone-like apatite was observed. The infrared spectroscopy of the precipitate in Figure 2 shows the typical spectrum of apatite. From the above, it is understood that when an alumina plate having calcium binding on the surface is soaked into a simulated body fluid, bone-like apatite is precipitated with a surface area rate of about 20% (the surface area of the aluminum plate being taken as 100%). Namely, the above alumina plate shows osteoconductivity.

For assessing the usefulness of the alumina plate having calcium binding on the surface as a medical tissue-binding material in more details, bone marrow cells obtained from SD rats of 4 weeks old were differentiated into osteoblast cells, and then the initial cell adhesion and cell growth were determined. The initial cell adhesion was 3302 ± 267 (± SD, bd) cells, the cell growth was 98333 ± 6244 cells and the ALP activity/total protein was 0.01425 ± 0.00072 (IUcm²/mg). As shown in Figure 4, the alumina plate having calcium binding on the surface indicates remarkable initial cell adhesion and cell growth and provides a medical tissue-binding material having a significant tissue affinity.

### Example 2

An alumina plate (Nikkato) as a ceramic material was soaked into a 50 mM aqueous solution of calcium chloride and treated under a hydrothermal condition at 125°C for 7 days. After washing sufficiently, the alumina plate was analyzed by XPS to confirm calcium binding on the surface as shown in Figure 1. The peak area was 3200 cps*eV. The calcium peak is larger than that in Example 1 in which a 10 mM solution of calcium chloride was used, from which it is understood that a higher amount of calcium bound to the alumina plate.

For assessing the osteoconductivity, the alumina plate having calcium binding on the surface was soaked into a simulated body fluid for 14 days. On the surface of the alumina plate, a precipitate considered to be bone-like apatite was observed. The infrared spectroscopy of the precipitate in Figure 2 shows the typical spectrum of apatite. From the above, it is understood that when an alumina plate having calcium binding on the surface is soaked into a simulated body fluid, bone-like apatite is precipitated with a surface area rate of about 20% (the surface area of the aluminum plate being taken as 100%). Namely, the above alumina plate shows osteoconductivity.

For assessing the usefulness of the alumina plate having calcium binding on the surface as a medical tissue-binding material in more details, bone marrow cells obtained from SD rats of 4 weeks old were differentiated into osteoblast cells, and then the initial cell adhesion and cell growth were determined. The initial cell adhering was 3611 ± 490 cells, the cell growth was 111230 ± 14045 cells and the ALP activity/total protein was 0.01863 ± 0.00052 (IUcm²/mg). As shown in Figure 4, the alumina plate having calcium binding on the surface shows remarkable initial cell adhesion and cell growth and provides a medical tissue-binding material having a significant tissue affinity. Both initial cell adhesion and cell growth are greater than those in Example 1.

### Example 3

An alumina plate (Nikkato) as a ceramic material was soaked into a 10 mM aqueous solution of calcium hydroxide and treated under a hydrothermal condition at 125°C for 7 days. After washing sufficiently, the alumina plate was analyzed by XPS to confirm calcium binding on the surface as shown in Figure 1. The peak area was 2600 cps*eV.

For assessing the osteoconductivity, the alumina plate having calcium binding on the surface was soaked in a simulated body fluid for 14 days. On the surface of the alumina plate, a precipitate considered to be bone-like apatite was observed. From the above, it is understood that when an alumina plate having calcium binding on the surface is soaked in a simulated body fluid, bone-like apatite is precipitated with a surface area rate of about 20% (the surface area of the aluminum plate being taken as 100%). Namely, the above alumina plate shows osteoconductivity.

For assessing the usefulness of the alumina plate having calcium binding on the surface as a medical tissue-binding material in more details, bone marrow cells obtained from SD rats of 4 weeks old were differentiated into osteoblast cells, and then the initial cell adhesion and cell growth were determined. The initial cell adhesion was 3425 ± 570 cells, the cell growth was 87562 ± 5923 cells and the ALP activity/total protein was 0.01573 ± 0.00092 (IUcm²/mg), Thus, the alumina base plate having calcium binding on the surface shows remarkable initial cell adhesion and cell growth and provides a medical tissue-binding material having a significant tissue affinity.

### Comparative Example 1

In order to show the usefulness of an alumina plate having calcium binding on the surface as a medical tissue-binding material, comparative test was carried out using an alumina plate confirmed to have no calcium binding on the surface by XPS analysis and give a peak area of 0 cps*eV.

The alumina plate was soaked into a simulated body fluid for 14 days as in Examples 1 and 2. No precipitate was found on the surface. Namely, the alumina plate was confirmed to show no osteoconductivity.

The initial cell adhesion and cell growth of the alumina plate having no calcium binding on the surface were determined. The alumina plate gave 1296 ± 185 cells in initial cell adhesion, 81605 ± 4908 cells in cell growth and 0.01078 ± 0.00098 IUcm²/mg in ALP activity/total protein. These values show the inferiority of the alumina plate to those in Examples 1 and 2.

### Comparative Example 2

In order to show the usefulness of an alumina plate having calcium binding on the surface as a medical tissue-binding material, comparative test was carried out using an alumina plate soaked into distilled water and treated under a hydrothermal condition at 125°C for 7 days. No calcium binding to the alumina plate was found by XPS analysis. The peak area was 0 cps*eV.

The alumina plate was soaked into a simulated body fluid for 14 days as in Examples 1 and 2. No precipitate was found on the surface. Namely, it was confirmed that the alumina plate treated under hydrothermal condition but having no calcium binding on the surface shows no osteoconductivity.

The initial cell adhesion and cell growth of the alumina plate having no calcium binding on the surface were determined. The alumina plate gave 1716 ± 266 cells in initial cell adhesion, 62639 ± 5297 cells in cell growth and 0.01437 ± 0.00206 IUcm²/mg in ALP activity/total protein. These values show the inferiority of the alumina plate to those in Examples 1 and 2.

The results of the medical tissue-binding materials in Examples 1 to 3 and Comparative Examples 1 to 2 are summarized in the table below.

**Table 2**

| | Base | Pretreatment of surface | Kind of Ca | Ca concentration (mmol/L) | Temperature of treatment | Period of treatment | XPS | SBF |
|---|---|---|---|---|---|---|---|---|
| Ex. 1 | Alumina | - | CaCl₂ | 10 | 125 °C | 7 days | O | 20% (14 days) |
| Ex.2 | Alumina | - | CaCl₂ | 50 | 125 °C | 7 days | O | 20% (14 days) |
| Ex. 3 | Alumina | - | Ca (CH₃COO)₂ | 10 | 125 °C | 7 days | O | 20% (14 days) |
| Comparative Ex. 1 | Alumina | - | - | - | - | - | X | 0% (14 days) |
| Comparative Ex. 2 | Alumina | - | - | 0 | 125 °C | - | X | 0% (14 days) |

### Example 4

A zirconia plate (Nikkato) as a ceramic material was soaked into a 10 mM aqueous solution of calcium chloride and treated under a hydrothermal condition at 125°C for 7 days. After washing sufficiently, the zirconia plate was analyzed by XPS to confirm calcium binding on the surface.

For assessing the osteoconductivity, the zirconia plate having calcium binding on the surface was soaked into a simulated body fluid for 14 days. On the surface of the zirconia plate, a precipitate considered to be bone-like apatite was observed. From the above, it is understood that when a zirconia plate having calcium binding on the surface is soaked into a simulated body fluid, bone-like apatite is precipitated with a surface area rate of about 100% (the surface area of the zirconia plate being taken as 100%). Namely, the above zirconia plate shows excellent osteoconductivity.

For assessing the usefulness of the zirconia plate having calcium binding on the surface as a medical tissue-binding material in more details, bone marrow cells obtained from SD rats of 4 weeks old were differentiated into osteoblast cells, and then the initial cell adhesion and cell growth were determined. The initial cell adhesion was 3333 ± 892 cells and the cell growth was 11014 ± 4127 cells. The zirconia base plate having calcium binding on the surface indicates remarkable initial cell adhesion and cell growth and provides a medical tissue-binding material having a significant tissue affinity.

### Comparative Example 3

In order to show the usefulness of a zirconia plate having calcium binding on the surface as a medical tissue-binding material, comparative test was carried out using a zirconia plate soaked into distilled water and treated under a hydrothermal condition at 125°C for 7 days. No calcium binding to the zirconia plate was found by XPS analysis.

The zirconia plate was soaked into a simulated body fluid for 14 days as in Example 4. No precipitate was found on the surface. Namely, it was confirmed that the zirconia plate shows no osteoconductivity.

The initial cell adhesion and cell growth of the zirconia plate having no calcium binding on the surface were determined. The zirconia plate gave 1482 ± 792 cells in initial cell adhesion and 8958 ± 3610 cells in cell growth.

These values show the inferiority of the zirconia plate to that in Example 4.

The results of the medical tissue-binding materials in Example 4 and Comparative Example 3 are summarized in the table below.

**Table 3**

| | Base | Pretreatment of surface | Kind of Ca | Ca concentration (mmol/L) | Temperature of treatment | Period of treatment | XPS | SBF |
|---|---|---|---|---|---|---|---|---|
| Ex. 4 | Zirconia | - | CaCl₂ | 20 | 125 °C | 7 days | O | 100% (14 days) |
| Comparative Ex. 3 | Zirconia | - | - | - | 125 °C | 7 days | X | 0% (14 days) |

### Example 5

A carbon plate as a ceramic material was soaked into a 10 mM aqueous solution of calcium chloride and treated under a hydrothermal condition at 200°C for 24 hours. After washing sufficiently, the carbon plate was analyzed by XPS to confirm calcium binding on the surface. The peak area was 1200 cps*eV.

For assessing the osteoconductivity, the carbon plate having calcium binding on the surface was soaked in a simulated body fluid for 14 days. On the surface of the carbon plate, a precipitate considered to be bone-like apatite was found by the electron microscopic observation and the infrared spectrum. Since the typical spectrum of apatite was observed, it is understood that when a carbon plate having calcium binding on the surface is soaked into a simulated body fluid, bone-like apatite is precipitated with a surface area rate of 1% (the surface area of the casrbon plate being taken as 100%). Namely, the above carbon plate shows osteoconductivity.

### Example 6

Ozone generated from an ozone generator (ED-OG-R4, Eco-design Co. Ltd.) was bubbled through distilled water at 50 °C to provide a saturated ozone solution. A carbon plate as a ceramic material was soaked into the ozone solution to ozonate, immersed in a 10 mM aqueous solution of calcium chloride and treated under a hydrothermal condition at 200°C for 24 hours. After washing sufficiently, the carbon plate was analyzed by XPS to confirm the peak of calcium. This result indicates that calcium bound to the carbon plate. The peak area was 1700 cps*eV.

For assessing the osteoconductivity, the carbon plate having calcium binding on the surface was soaked into simulated body fluid for 14 days. On the surface of the carbon plate to which calcium binds, a precipitate considered to be bone-like apatite was found by the electron microscopic observation and the infrared spectrum. Since the typical spectrum of apatite was observed, it is understood that when a carbon plate having calcium binding on the surface is soaked into a simulated body fluid, bone-like apatite is precipitated with a surface area rate of 3% (the surface area of the carbon platl being taken as 100%). Namely, the above carbon plate shows osteoconductivity. The tissue-binding ability is greater than that of Example 5.

### Comparative Example 4

In order to show the usefulness of a carbon plate having calcium binding on the surface as a medical tissue-binding material, comparative test was carried out using a carbon plate soaked into distilled water and treated under a hydrothermal condition at 200°C for 24 hours. No calcium binding to the carbon plate was found by XPS analysis. The peak area was 0 cps*eV.

The carbon plate was soaked into a simulated body fluid for 14 days as in Example 4. No precipitate was found on the surface. Namely, it was confirmed that the carbon plate shows no osteoconductivity.

The results of the medical tissue-binding materials in Examples 5 and 6 and Comparative Example 4 are summarized in the table below.

**Table 4**

| | Base | Pretreatment of surface | Kind of Ca | Ca concentration (mmol/L) | Temperature of treatment | Period of treatment | XPS | SBF |
|---|---|---|---|---|---|---|---|---|
| Ex. 5 | Carbon | - | CaCl₂ | 10 | 200 °C | 1 day | O | 1% (14 days) |
| Ex. 6 | Carbon | Ozone | CaCl₂ | 10 | 200 °C | 1 day | O | 3% (14 days) |
| Comparative Ex. 4 | Carbon | - | - | - | 200 °C | - | X | 0% (14 days) |

### Example 7

A hydroxyapatite plate as a ceramic material was soaked into a 10 mM aqueous solution of calcium chloride and treated under a hydrothermal condition at 200°C for 24 hours. After washing sufficiently, the hydroxyapatite plate was analyzed by XPS to detect the peaks of calcium and phosphorus. The peak ratio of calcium to phosphorus of the hydroxapatite plate as treated above was larger than that before the treatment. Namely, the peak area ratio of calcium to phosphoric acid on the untreated hydroxyapatite plate was 2.557, while that on the hydroxyapatite plate treated with a 100 mM aqueous solution of calcium chloride at 200°C for 1 day was 2.373. From the above results, it is understood that calcium binds to the surface of a hydroxyapatite plate by treating with a 100 mM aqueous solution of calcium chloride at 200°C for 1 day.

For assessing the osteoconductivity, the hydroxcyapatite plate having calcium binding on the surface was soaked in a simulated body fluid for 14 days. On the surface, a precipitate considered to be bone-like apatite was found with a surface area rate of about 5% (the surface area of the base material being taken as 100%) by the electron microscopic observation and the infrared spectrum. The amount of the precipitate on the hydroxyapatite plate treated with the calcium chloride solution was larger than that of the untreated plate when those were soaked a into simulated body fluid. Consequently, a hydroxyapatite plate having calcium binding on the surface is greater than that having no calcium binding in bone-binding ability.

### Comparative Example 5

Comparative test was carried out to show the usefulness of the medical tissue-binding material of an apatite plate to which calcium binds. An apatite plate was soaked into distilled water and treated under hydrothermal condition at 200 °C for 24 hours. No increase of calcium peak area was found in the apatite plate treated under hydrothermal condition comparing with the untreated one by XPS analysis.

In order to show the usefulness of an apatite plate having calcium binding on the surface as a medical tissue-binding material, comparative test was carried out using an apatite plate soaked into distilled water and treated under a hydrothermal condition at 200°C for 24 hours. No increase of the peak area of calcium in comparison with that of the untreated apatite plate was observed by XPS analysis.

The apatite plate was soaked into a simulated body fluid for 14 days as in Example 7. On the surface, a precipitate was observed with a surface area rate of about 4% (the surface area of the aplate plate being taken as 100%). Consequently, it is understood that the bone-binding ability of the apatite plate treated under a hydrothermal condition is inferior to that of the apatite plate having calcium binding on the surface in Example 7.

The results of the medical tissue-binding materials in Example 7 and Comparative Example 5 are summarized in the table below.

**Table 5**

| | Base | Pretreatment of surface | Kind of Ca | Ca concentration (mmol/L) | Temperature of treatment | Period of treatment | XPS | SBF |
|---|---|---|---|---|---|---|---|---|
| Ex. 7 | Apatite | - | CaCl₂ | 10 | 200 °C | 1 day | Separately | 5% (14 days) |
| Comparative Ex. 5 | Apatite | - | - | - | 200 °C | - | Separately | 4% (14 days) |

The medical tissue-binding materials of Example 8 and Comparative Example 6 were prepared by using silica as the base material under the conditions below according to the method as described in Examples and Comparative Examples above. The results are summarized in the table below. The peak areas of Example 8 and Comparative Example 6 were 6600 cps*eV and 0 cps*eV, respectively.

**Table 6**

| | Base | Pretreatment of surface | Kind of Ca | Ca concentration (mmol/L) | Temperature of treatment | Period of treatment | XPS | SBF |
|---|---|---|---|---|---|---|---|---|
| Ex. 8 | Silica | - | CaCl₂ | 50 | 125 °C | 7 days | O | 80% (7 days) |
| Comparative Ex. 6 | Silica | - | - | - | 125 °C | 7 days | X | - |

### Example 9

A polyimide film as a polymer material was soaked into a 10 mM aqueous solution of calcium chloride and treated under a hydrothermal condition at 140°C for 14 days. After washing sufficiently, the polyimide film was analyzed by XPS to confirm calcium binding on the surface. The peak area was 1000 cps*eV.

For assessing the bone-binding ability, the polyimide film having calcium binding on the surface was soaked into a simulated body fluid for 14 days. On the surface, a precipitate considered to be bone-like apatite was observed. Since the typical spectrum was observed, it is understood that when a polyimide film having calcium binding on the surface is soaked into simulated body fluid, a bone-like apatite is precipitated with a surface area rate of about 20% (the surface area of the base film being taken as 100%). Namely, the above polyimide film shows osteoconductivity.

### Example 10

A polyimide film as a polymer material was soaked into a 10 mM aqueous solution of calcium chloride and treated under a hydrothermal condition at 90°C for 14 days. After washing sufficiently, the polyimide film was analyzed by XPS to confirm calcium binding on the surface. The peak area was 1300 cps*eV. The amount of calcium binding on the surface was smaller that that of the polyimide film treated in Example 9.

For assessing the bone-binding ability, the polyimide film having calcium binding on the surface was soaked into a simulated body fluid for 14 days. On the surface, a precipitate considered to be bone-like apatite was observed with a surface area rate of about 2 % (the surface area of the base film being taken as 100 %). The amount of the precipitate was smaller in comparison with that of the polyamide film treated in Example 9. From the above results, it is understood that the extent of the bone binding ability can be controlled by changing the treating conditions such as temperature.

### Example 12

For sulfonation, a polyimide film was soaked into conc. sulfuric acid at 100°C for 6 hours. No difference was seen between the treated and untreated polyimide films on the scanning electron microscope observation. In the FT-IR analysis for the sulfonated polyimide film, the absorption of a sulfonate group was seen at 1207 cm⁻¹ and 1128 cm⁻¹.

The sulfonated polyimide film was soaked into a 10 mM aqueous solution of calcium chloride and subjected to treatment under a hydrothermal condition at 140°C for 1 day. After washing sufficiently, the resultant polyimide film was analyzed by XPS to confirm calcium binding on the surface. The peak area was 1350 cps*eV.

For assessing the bone-binding ability, the sulfonated Polyimide film having calcium binding on the surface was soaked into a simulated body fluid for 14 days. On the surface, a precipitate considered to be bone-like apatite was observed. Since the precipitate gave the typical spectrum of apatite, it is understood that when a sulfonated Polyimide film having calcium binding on the surface is soaked into a simulated body fluid, bone-like apatite is precipitated with a surface area rate of 60% (the surface area of the polyimide film being taken as 100%). Namely, the above sulfonated polyimide film shows osteoconductivity.

### Example 13

A chloromethylated polyimide film was aminated by soaking into a solution of a 30% aqueous solution of trimethylamine at 70°C for 24 hours. No difference was seen between the treated and untreated polyimide films on the scanning electron microscope observation. In the FT-IR analysis for the aminated polyimide film, the absorption of an amino group was seen at 1604 cm⁻¹ and 1525 cm⁻¹.

The aminated polyimide film was soaked into a 10 mM aqueous solution of calcium chloride and subjected to treatment under a hydrothermal condition at 140°C for 1 day. After washing sufficiently, the resultant polyimide film was analyzed by XPS to confirm calcium binding on the surface. The peak area was 1400 cps*eV.

For assessing the bone-binding ability, the aminated polyimide film having calcium binding on the surface was soaked into a simulated body fluid for 14 days. On the surface, a precipitate considered to be bone-like apatite was observed. Since the precipitate gave the typical spectrum of apatite, it is understood that when an aminated polyimide film having calcium binding on the surface is soaked into a simulated body fluid, bone-like apatite is precipitated with a surface area rate of about 50% (the surface area of the aminated polyimide film being taken as 100%). Namely, the above aminated polyimide film shows osteoconductivity.

### Example 14

A polyimide film was soaked into a 5% solution of propyltriethoxysilane isocyanate in tetrahydrofuran (THF) at 40°C for 24 hours. The polyimide film was then washed with THF and dried in air. No difference was seen between the treated and untreated polyimide films on the scanning electron microscope observation. Silicon was found in the XPS analysis.

The silanolated polyimide film was soaked into a 10 mM aqueous solution of calcium chloride and subjected to treatment under a hydrothermal condition at 140°C for 1 day. After washing sufficiently, the resultant polyimide film was analyzed by XPS to confirm calcium binding on the surface.

For assessing the bone-binding ability, the silanolated polyimide film having calcium binding on the surface was soaked into a simulated body fluid for 14 days. On the surface, a precipitate considered to be bone-like apatite was observed. Since the precipitate gave the typical spectrum of apatite, it is understood that when a silanolated polyimide film having calcium binding on the surface is soaked into a simulated body fluid, bone-like apatite is precipitated with a surface area rate of about 50% (the surface area of the silanolated polyimide film being taken as 100%). Namely, the above silanolated polyimide film shows osteoconductivity.

### Comparative Example 7

Comparative test was carried out to show the usefulness of the medical tissue-binding material of a polyimide film having calcium binding on the surface. A polyimide film was soaked into distilled water and treated under hydrothermal condition at 140°C for 24 hours. No increase of the peak area of calcium was observed on the polyimide film treated as above in comparison with the untreated one by XPS analysis.

The polyimide film was soaked into a simulated body fluid for 14 days as in Example 7. On the surface, no precipitate was observed. It was thus understood that the polyimide film treated under a hydrothermal condition has no bone binding ability.

The medical tissue-binding materials of Examples 11 and 15 were prepared in the same manner as Examples above under the conditions in the table below. The assessment of the medical tissue-binding materials of Examples 9 to 15 and Comparative Example 7 are summarized in the table below. The peak areas of Example 11 and 15 were 1300 cps*eV and 1400 cps*eV, respectively.

**Table 7**

| | Base | Pretreatment of surface | Kind of Ca | Ca concentration (mmol/L) | Temperature of treatment | Period of treatment | XPS | SBF |
|---|---|---|---|---|---|---|---|---|
| Ex. 9 | Polyimide | - | CaCl₂ | 10 | 140 °C | 14 days | ○ | 20% (14 days) |
| Ex. 10 | Polyimide | - | CaCl₂ | 10 | 90 °C | 14 days | ○ | 2% (14 days) |
| Ex. 11 | Polyimide | Hydroxyl group | CaCl₂ | 10 | 140 °C | 1 day | ○ | 40% (14 days) |
| Ex. 12 | Polyimide | Sulfonic group | CaCl₂ | 10 | 140 °C | 1 day | ○ | 60% (14 days) |
| Ex. 13 | Polyimide | Amino group | CaCl₂ | 10 | 140 °C | 1 day | ○ | 50% (14 days) |
| Ex. 14 | Polyimide | Silanol group | CaCl₂ | 10 | 140 °C | 1 day | ○ | 50% (14 days) |
| Ex. 15 | Polyimide | Phosphate group | CaCl₂ | 10 | 140 °C | 1 day | ○ | 70% (14 days) |
| Comparative Ex. 7 | Polyimide | - | - | - | 140 °C | 1 day | X | 0% (14 days) |

### Example 16

A silk sheet as a polymer material was soaked into a 10 mM aqueous solution of calcium chloride and treated under a hydrothermal condition at 125°C for 7 days. After washing sufficiently, the silk sheet analyzed by XPS to confirm calcium binding on the surface.

For assessing bone-binding ability, the silk sheet having calcium binding on the surface was soaked into a simulated body fluid for 14 days. On the surface, a precipitate considered to be bone-like apatite was observed. From the above, it is understood that when a silk sheet having calcium binding on the surface is soaked into a simulated body fluid, bone-like apatite is precipitated with a surface area rate of about 10% (the surface area of the silk sheet being as 100%). Namely, the above silk sheet shows osteroconductivity.

### Comparative Example 8

In order to show the usefulness of a silk sheet having calcium binding on the surface as a medical tissue-binding material, comparative test was carried out using distilled water. XPS analysis revealed no calcium binding on the surface.
The silk sheet was soaked into simulated body fluid for 14 days. On the surface, no precipitate was observed. The silk sheet was thus confirmed to show no osteoconductivity.

The results of the medical tissue-binding materials of Example 16 and Comparative Example 8 are summarized in the table below.

**Table 8**

| | Base | Pretreatment of surface | Kind of Ca | Ca concentration (mmol/L) | Temperature of treatment | Period of treatment | XPS | SBF |
|---|---|---|---|---|---|---|---|---|
| Ex. 16 | Silk | - | CaCl₂ | 10 | 125 °C | 7 days | ○ | 10% (14 days) |
| Comparative Ex. 8 | Silk | - | - | - | 150 °C | 7 days | X | 0% (14 days) |

The medical tissue-binding materials of Example 17 and Comparative Example 9 were prepared by using polyethylene terephthalate as the base material in the same manner as Examples and Comparative Examples above under the conditions in the table below. The results are summarized in the table below.

**Table 9**

| | Base | Pretreatment of surface | Kind of Ca | Ca concentration (mmol/L) | Temperature of treatment | Period of treatment | XPS | SBF |
|---|---|---|---|---|---|---|---|---|
| Ex. 17 | Polyethylene terephthalate | - | CaCl₂ | 10 | 140 °C | 1 day | ○ | 2% (14 days) |
| Comparative Ex. 9 | Polyethylene terephthalate | - | - | - | 150 °C | 1 day | X | 0% (14 days) |

The medical tissue-binding materials of Example 18 and Comparative Example 10 were prepared by using silicone as the base material in the same manner in Examples and Comparative Examples above under the conditions in the table below. The results are summarized in the table below.

**Table 10**

| | Base | Pretreatment of surface | Kind of Ca | Ca concentration (mmol/L) | Temperature of treatment | Period of treatment | XPS | SBF |
|---|---|---|---|---|---|---|---|---|
| Ex. 18 | Silicone | - | CaCl₂ | 10 | 200 °C | 1 day | ○ | 1% (14 days) |
| Comparative Ex. 10 | Silicone | - | - | - | 200 °C | 1 day | X | 0% (14 days) |

### Example 19

A stainless steel plate as a metal material was soaked into a 50 mM aqueous solution of calcium chloride and treated under a hydrothermal condition at 200°C for 24 hours. After washing sufficiently, the stainless steel plate was analyzed by XPS to confirm calcium binding on the surface. The peak area was 400 cps*eV.

For assessing the osteoconductivity, the stainless steel plate having calcium binding on the surface was soaked into a simulated body fluid for 7 days. On the surface, a precipitate considered to be bone-like apatite was observed with a surface area rate of about 80% (the surface area of the stainless steel plate being taken as 100%). Therefore, the above stainless steel plate shows osteoconductivity.

### Comparative Example 11

In order to show the usefulness of a stainless steel plate having calcium binding on the surface as a medical tissue-binding material, comparative test was carried out by using distilled water. No calcium binding to the stainless steel plate was found by XPS analysis. The peak area was 0 cps*eV.

The stainless steel plate was soaked into a simulated body fluid for 7 days. No precipitate was found on the surface. Therefore, the above stainless steel was confirmed to show no osteoconductivity.

The results of the medical tissue-binding material of Examples 19 and Comparative Example 11 are summarized in the table below.

**Table 11**

| | Base | Pretreatment of surface | Kind of Ca | Ca concentration (mmol/L) | Temperature of treatment | Period of treatment | XPS | SBF |
|---|---|---|---|---|---|---|---|---|
| Ex. 19 | Stainless steel | - | CaCl₂ | 10 | 200 °C | 1 day | ○ | 80% (7 days) |
| Comparative Ex. 11 | Stainless steel | - | - | - | 200 °C | 1 day | X | 0% (7 days) |

The medical tissue-binding materials of Example 20 and Comparative Example 12 were prepared by using a cobalt chrome plate as the base material in the same manner as Examples and Comparative Examples above under the conditions in the table below. The results are summarized in the table below. The peak area of Example 20 was 400 cps*eV.

**Table 12**

| | Base | Pretreatment of surface | Kind of Ca | Ca concentration (mmol/L) | Temperature of treatment | Period of treatment | XPS | SBF |
|---|---|---|---|---|---|---|---|---|
| Ex. 20 | Cobalt chrome | - | CaCl₂ | 10 | 200 °C | 1 day | ○ | 2% (14 days) |
| Comparative Ex. 12 | Cobalt chrome | - | - | - | 200 °C | 1 day | X | 0% (14 days) |

As illustrated above, the present invention provides a medical tissue-binding material which shows 1) no tissue injury, 2) osteoconductivity, 3) replaceability for bone and 4) mechanical strength required for prosthesis.

## Claims

1. A medical tissue-binding material which comprises a base material having calcium binding to the surface, provided that the base material is not titanium or titanium alloy.

2. The medical tissue-binding material according to claim 1, wherein the base material is a ceramics.

3. The medical tissue-binding material according to claim 1, wherein the base material is a polymer.

4. The medical tissue-binding material according to claim 1, wherein the base material is a metal.

5. A method for preparing the medical tissue-binding material according to any one of claims 1 to 4, which comprises a step of soaking a base material into a calcium ion containing solution.

6. The method according to claim 5, wherein the soaking is carried out under a hydrothermal condition.

7. The method according to claim 5 or 6, wherein at least one group selected from the group consisting of hydroxyl, carboxyl, sulfonate, amino, silanol and phosphate is introduced to the surface of the base material before or simultaneously with the soaking.

8. A method for preparing a medical tissue-binding material comprising a base material having calcium binding on the surface, said base material being selected from the group consisting of titanium and titanium alloy, which comprises introducing at least one group selected from the group consisting of hydroxyl, carboxyl, sulfonate, amino, silanol and phosphate to the surface of the base material and soaking the base material into a calcium ion containing solution, the introducing and the soaking being carried out in order or simultaneously.
